(19) Europäisches Patentamt
European Patent Office
Office européen des brevets

(11) Publication number: 0 075 626
A1

# (12) EUROPEAN PATENT APPLICATION

(21) Application number: 81304492.2

(22) Date of filing: 29.09.81

(51) Int. Cl.³: C 07 H 17/06
A 61 K 31/70

(43) Date of publication of application: 06.04.83 Bulletin 83/14

(84) Designated Contracting States: BE IT

(71) Applicant: AMERICAN CYANAMID COMPANY
Berdan Avenue
Wayne New Jersey 06904(US)

(72) Inventor: Nair, Vijay Gopalan
1090 Amsterdam Avenue Apt. 15D
New York, N.Y. 10025(US)

(72) Inventor: Bernstein, Seymour
26 Scott Drive
New City New York 10956(US)

(74) Representative: Allam, Peter Clerk et al,
LLOYD WISE, TREGEAR & CO. Norman House 105-109 Strand
London WC2R 0AE(GB)

## (54) Novel rutin nona- and deca(H-)sulfate salts, methods of preparing novel salts, and use of novel salts of complement inhibitors.

(57) The invention provides novel rutin poly(H-) sulfates of the general formula:

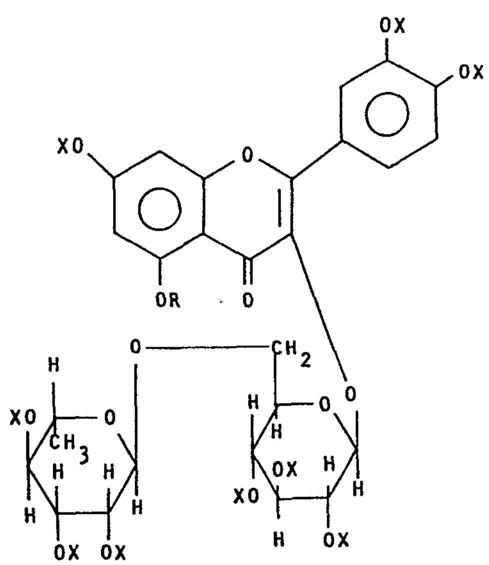

wherein R is hydrogen or $-SO_3A$; each X is $-SO_3A$; and A is a pharmaceutically acceptable cation. These rutin nona- and deca(H-)sulfate sodium salts possess unexpectedly high in vitro activity as complement inhibitors.

EP 0 075 626 A1

# NOVEL RUTIN NONA- AND DECA(H-)SULFATE SALTS, METHODS OF PREPARING NOVEL SALTS, AND USE OF NOVEL SALTS AS COMPLEMENT INHIBITORS

## BACKGROUND OF THE INVENTION

This invention relates to novel rutin poly(H-)-sulfate salts which possess very good activity as inhibitors of the complement system of warm-blooded animals.

The term "complement" refers to a complex group of proteins in body fluids that, working together with antibodies or other factors, play an important role as mediators of immune, allergic, immunochemical and/or immunopathological reactions. The reactions in which complement participates take place in blood serum or in other body fluids, and hence are considered to be humoral reactions. A detailed discussion of the complement system is given in our European Patent Application No. 13470A (Serial No. 79302349.0) incorporated herein by reference.

In our European Patent Application No. 13470A, we have disclosed that rutin poly(H-)sulfate salts of the general formula (I) below interact with the complement reaction sequence, thereby inhibiting complement activity in body fluids:

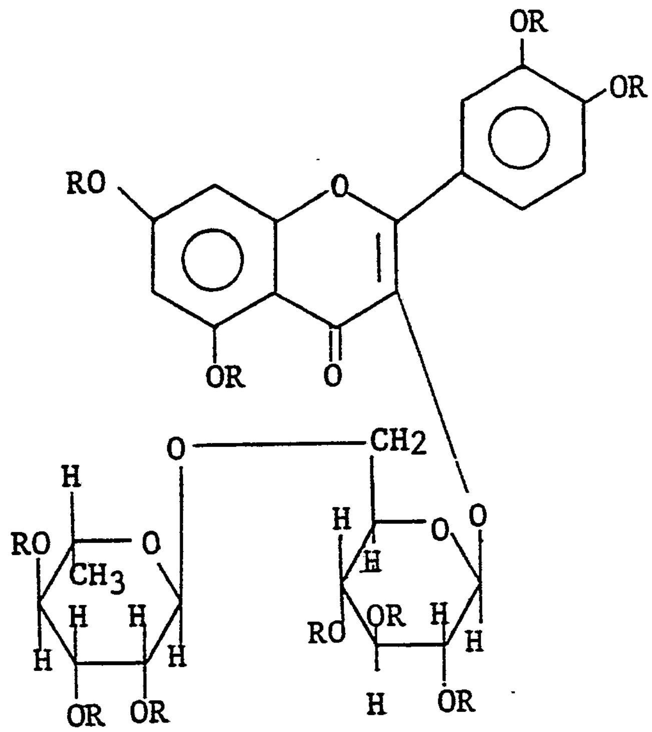

(I)

wherein each R is selected from the group consisting of hydrogen and $-SO_3A$; wherein A is a pharmaceutically acceptable salt cation with the proviso that at least six of the R groups are $-SO_3A$.

A process for the preparation of the rutin poly(H-)sulfate alkali metal salts within the scope of formula (I) above is also taught. In the process rutin, or so-called "rutin water-soluble" which is a rutin sulfate sodium salt commercially available from E. Merck, Darmstadt, West Germany, Catalogue No. 500014, is reacted in a suitable solvent with a trialkylamine-sulfur trioxide complex at $50^o$-$90^oC$, and the resulting trialkylamine sulfate salt is isolated and then treated with an alkali metal acetate in aqueous solution. Examples of this process are given in which rutin or rutin water soluble is reacted, in dimethylformamide, with a trimethylamine-sulfur trioxide complex at $65^o$-$70^oC$, the resulting trimethylamine salt is isolated from absolute ethanol at room temperature, and thereafter reacted with aqueous sodium acetate solution to give a product identified as "rutin poly(H-)sulfate, sodium salt" which has a sulfur analysis varying between 15.3 and 16.5%.

By analysis it has been subsequently shown by us that this product contains approximately 85% rutin nona- and deca(H-) sulfate sodium salts in approximately equal amounts, the remaining 15% being a mixture of the hexa- through octa(H-) sulfate sodium salts of rutin. This mixture is also shown to possess activity as a complement inhibitor, in contrast to rutin water-soluble which shows no activity by the tests employed.

## SUMMARY OF THE INVENTION

It has now been found that by modifying the process for preparing rutin poly(H-)sulfate salts taught in European Application No. 13470A, supra, in certain specific ways, described below, it is possible to prepare rutin nona(H-)sulfate salts which are at least substantially free of the corresponding deca(H-)salt, and similarly rutin deca(H-)sulfate salts which are at least substantially free of the corresponding nona(H-)salt. Most unexpectedly, we have found that the substantially pure nona- and deca- salts which are thereby obtained possess significantly better activity in complement-inhibiting tests than the mixtures of hexa- through deca- salts which are prepared by the Examples of European Application No. 13740A. This result is very surprising since it would not have been thought that two of the five components of the previously described mixtures would each individually have much greater activity than a mixture consisting of all five components.

By the expression "at least substantially free", and similar expressions employed herein, we mean to refer to a rutin nona-(or deca-) salt which contains less than 10% by weight of the corresponding deca-(or nona-) salt. In fact, by means of the novel processes of this invention it is possible to obtain the nona- and deca- salts which are about 95% or better pure.

The rutin deca(H-)sulfate salts of the present invention are novel compounds which have the general formula

(II):

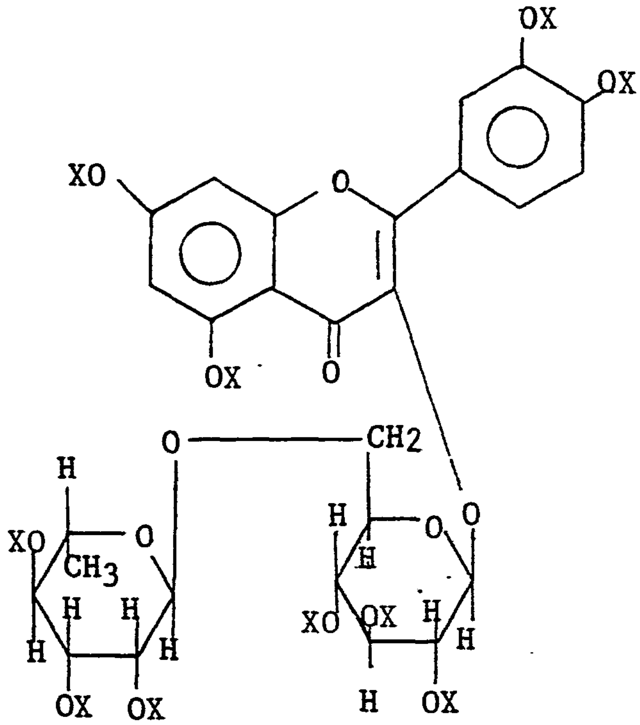

(II)

In general formula (II), each X is $-SO_3A$, wherein A is a pharmaceutically acceptable cation, preferably an alkali metal, most preferably sodium.

The rutin deca(H-)sulfate salts may be prepared substantially free of the corresponding nona- salts by a process which comprises the steps of:

(1) reacting rutin or a rutin poly(H-)sulfate having from one to nine sulfates in a suitable solvent with a triethylamine-sulfur trioxide complex at $50^{\circ}$-$90^{\circ}C$;

(2) isolating the resulting triethylamine sulfate salt from acetone containing amine and cooled to below room temperature; and

(3) treating the isolated salt from step (2) with a pharmaceutically acceptable salt cation in aqueous solution.

In this process the use of a triethylamine-sulfur trioxide complex as the sulfating agent is critical to obtaining the desired pure deca- salt. It is not yet known why this should be so, but in practice it is found that employing a trimethylamine-sulfur trioxide complex as taught

in our European Application No. 13470A, supra, always leads to a mixture of the hexa- through deca- salts. The solvent employed for the sulfating reaction may be dimethylformamide, for example, but in accordance with another feature of our process we have found that N,N-dimethylacetamide gives a somewhat purer product, and this therefore is the preferred solvent for use herein.

Another critical step in our novel process for preparing substantially pure rutin deca(H-)sulfate salts consists of the isolation procedure. Thus, it is found that the triethylamine-sulfate salt should be isolated from acetone which contains quantities of an amine, preferably a tertiary amine, sufficient to maintain the medium at a basic pH, preferably about pH 8-9, although a higher pH is acceptable. Preferably this amine is triethylamine. This isolation should be effected at low temperatures, suitably about 0°C. Again, it is not known for certain why these isolation procedures should be critical, but from experience we have found that they are necessary in our process.

The novel rutin nona-(H-)sulfate salts of our invention may be represented by the general formula (III):

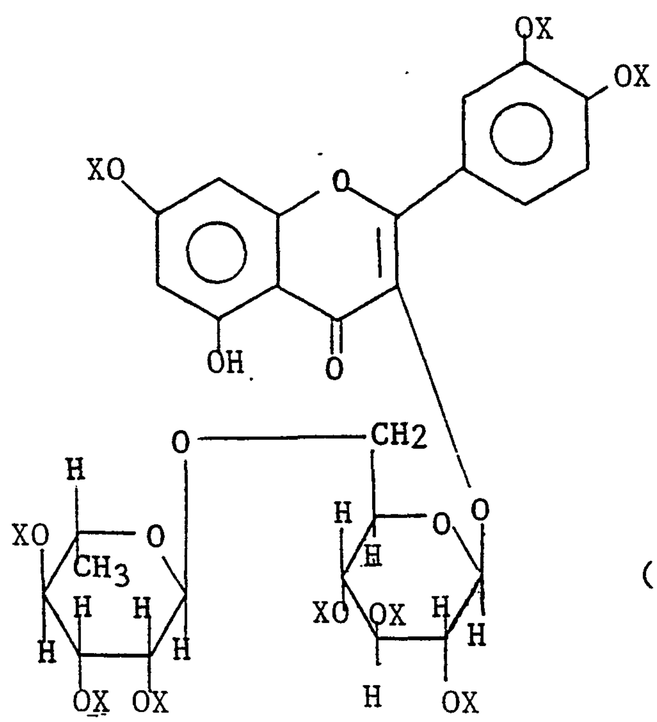

(III)

In formula (III), each X is as already defined.

The rutin nona(H-)sulfate salts of formula (III) may be prepared substantially free of the corresponding rutin deca(H-)sulfate salts by a process which comprises the steps of:

(1) reacting rutin or a rutin poly(H-)sulfate having from one to eight sulfates in a suitable solvent with a triethylamine-sulfur trioxide complex at $50^{o}$-$90^{o}C$;

(2) isolating the resulting triethylamine sulfate salt from acetone at ambient temperatures; and

(3) treating the isolated salt from step (2) with a pharmaceutically acceptable salt cation in aqueous solution.

This process for the preparation of the substantially pure nona- salts differs from the process for preparing the substantially pure deca- salts described above essentially in the isolation procedures employed. Thus, the sulfation step (1) again requires the use of triethylamine-sulfur trioxide complex as the sulfating agent, and N,N-dimethylacetamide is again the preferred solvent medium for the reaction. The isolation procedure also uses acetone, but in contrast to the process for making the deca- salts the acetone should not contain an added amine. Furthermore, it is important that the isolation should be effected at ambient temperatures (around $20^{o}$-$30^{o}C$, preferably $25^{o}C$).

In both processes of this invention the triethylamine sulfate salt isolated from the acetone solution is then treated with a pharmaceutically acceptable salt cation selected from the group consisting of alkali metals, alkaline earth metals, and heavy metals to yield the corresponding rutin sulfate salt. For this reaction the use of alkali metal acetates is preferred; particularly preferred is sodium acetate.

Examples of the preparation of the novel rutin poly(H-)sulfate salts of this invention will now be given by way of illustration only.

## Example 1

## Rutin deca (H-sulfate) Sodium Salt

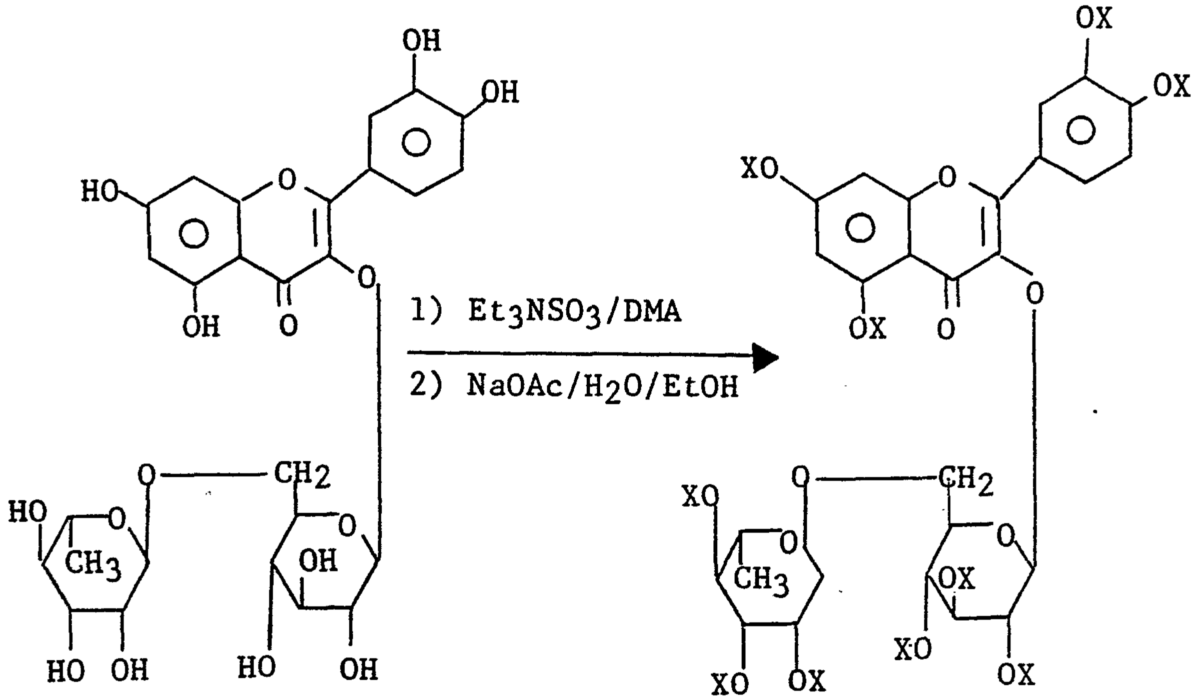


$C_{27}H_{30}O_{16}$

M. wt. 610.53

X = $SO_2ONa$

$C_{27}H_{27}O_{46}S_{10}Na_{10}$

M. wt. 1630.93

Triethylamine-sulfur trioxide (70.2 g, 0.388 mole) is dissolved in N,N-dimethylacetamide (245 ml) and Drierite (49 g) is added to it. The mixture is heated at 63-65° for 20 minutes. Rutin (5.95 g, 9.75 mmoles) is added to the mixture with swirling and heating continued at 63-65° for 3 hours. The reaction mixture is then rapidly cooled and filtered into acetone (2.5 ℓ) containing triethylamine (15 ml). The oil that separate is allowed to settle down overnight at ~0°. The supernatant liquid is decanted off and the oil washed several times with acetone to remove any excess triethylamine-sulfur trioxide.

The oily product is dissolved by the addition of sodium acetate solution (30%, 40 ml) and distilled water (50 ml). The solution is allowed to stand at r.t. for 20 minutes and then filtered. The filtrate is added gradually into ethanol (2.3 ℓ) with vigorous stirring. The stirring is continued for ~40 minutes. The granular product is filtered, washed repeatedly with ethanol and then with ether, and then dried in vacuo at r.t. for 18 hours. 14.5 g. (91%) off-white powder is obtained.

Calcd for $C_{27}H_{20}O_{46}Na_{10}S_{10}$ (1630.93)

C = 19.88, H = 1.24, S = 19.66, Na = 14.10

Found C = 18.98, H = 2.05, S = 18.25, Na = 14.52

KF = 2.58%

By high-pressure liquid chromatography analysis, the product contains 95% ± 1% of rutin deca(H-)sulfate, sodium salt and only 5% ± 1% rutin nona(H-)sulfate, sodium salt.

Example 2

Rutin nona (H-sulfate) Sodium Salt

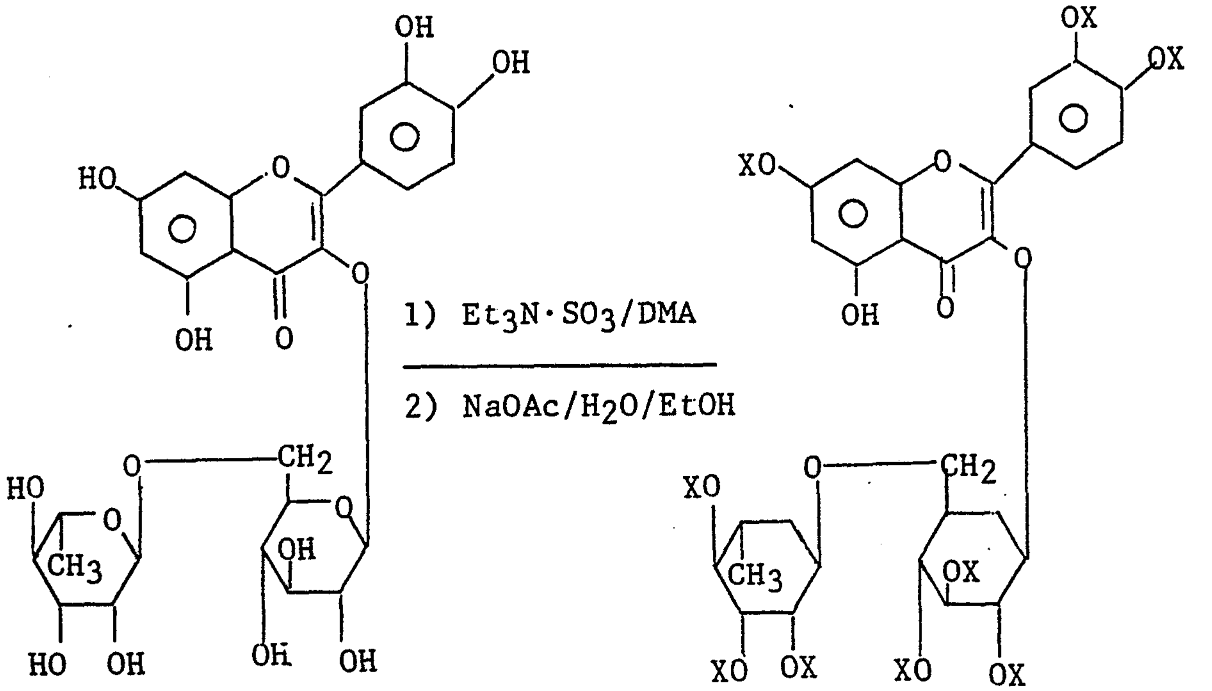


$C_{27}H_{30}O_{16}$
M. wt. 610.53

X = $SO_2ONa$
$C_{27}H_{21}O_{43}S_9Na_9$
M. wt. 1528.61

Triethylamine-sulfur trioxide (70.2 g, 0.388 mole) is dissolved in N,N-dimethylacetamide (245 ml) and Drierite (49 g) is added to it. The mixture is heated at 63-65° for 20 minutes. Rutin (5.95 g. 9.75 mmoles) is added to the mixture with swirling and heating continued at 63-65° for 3 hours. The reaction mixture is then rapidly cooled and filtered into acetone (2.5 ℓ). The oil that separated is allowed to settle down overnight at r.t. The supernatant liquid is decanted off and the oil washed several times with acetone to remove any excess triethylamine-sulfur trioxide.

The oily product is dissolved by the addition of sodium acetate solution (30%, 40 ml) and distilled water (50 ml). The solution is allowed to stand at r.t. for

20 minutes and then filtered. The filtrate is added gradually into ethanol (2.3 ℓ) with vigorous stirring. The stirring is continued for ~ 40 minutes. The granular product is filtered, washed repeatedly with ethanol and then with ether, and then dried in vacuo at r.t. for 18 hours. 12.8 g (86%) yellow powder is obtained.

Clcd for $C_{27}H_{21}O_{43}Na_9S_9$ (1528.61)

C = 21.21, H = 1.38, S = 18.87, Na = 13.58

Found C = 19.87, H = 2.61, S = 17.33, Na = 13.78

KF = 3.37%

By high-pressure liquid chromatography analysis, the product contains 95% ± 1% rutin nona(H-)sulfate, sodium salt and only 5% ± 1% rutin deca(H-)sulfate sodium salt.

The novel compounds of this invention show surprisingly high complement inhibiting activity as demonstrated by the Test Code 026 (C 1 inhibitor) which measures the ability of activated complement protein C1 to destroy fluid phase human complement portein C2 in the presence of complement protein C4 at appropriate dilutions of the test compound. An active inhibitor protects C2 from C1 and C4. The results of this test on the compounds of this invention, together with the results of the same test on a mixture of rutin nona(H-)sulfate, sodium salt and rutin deca(H-)sulfate, sodium salt prepared in accordance with the teachings of Example 2 of European Application No. 13470A, supra, are given below:

| Compound | In Vitro Activity Test Code 026 |
|---|---|
| *Rutin poly(H-)sulfate sodium salt (Analysis S=15.3%) | +7 |
| Rutin deca(H-)sulfate sodium salt | +11 |
| Rutin nona(H-)sulfate sodium salt | +10 |

* comparative test on mixture

This test measures activity in wells by a serial dilution assay. The higher the well number the higher is the activity. The serial dilutions are two-fold, so that in the test shown above the rutin deca(H-)sulfate sodium salt in accordance with this invention is 16 times as active as the mixture of hexa- through deca- salts produced by Example 2 of European Application No. 13470A, whilst the rutin nona(H-)sulfate sodium salt in accordance with this invention is 8 times as active as the aforesaid mixture. These results are very surprising, and important, and could in no way have been predicted from the disclosure of the said European Application.

The rutin poly(H-)sulfate salts of the present invention may be administered internally, e.g. orally, or parenterally, e.g. intra-articularly, to a warm-blooded animal, to inhibit complement in the body fluid of the animal, such inhibition being useful in the amelioration or prevention of those reactions dependent upon the function of complement, such as inflammatory process and cell membrane damage induced by antigen-antibody complexes. A range of doses may be employed depending on the mode of administration, the condition being treated and the particular compound being used. For example, for intravenous or subcutaneous use from about 5 to about 50 mg/kg/day, or every six hours for more rapidly excreted salts, may be used. For intra-articular use for large joints such as the knee, from about 2 to about 20 mg/joint per week may be used with proportionally smaller doses for smaller joints. The dosage range is to be adjusted to provide optimum therapeutic response in the warm-blooded animal being treated. In general, the amount of compound administered can vary over a wide range to provide from about 5 mg/kg to about 100 mg/kg of body weight of animal per day. The usual daily dosage for a 70 kg subject may vary from about 350 mg to about 3.5 g. Unit doses of the acid or salt can contain from about 0.5 mg to about 500 mg.

The rutin poly(H-)sulfate salts of the present

invention may also be administered topically in the form of ointments, creams, lotions and the like, suitable for the treatment of complement dependent dermatological disorders.

Moreover, the rutin poly(H-)sulfate salts of the present invention may be administered in the form of dental pastes, ointments, buccal tablets and other compositions suitable for application periodontally for the treatment of periodontitis and related diseases of the oral cavity.

In therapeutic use, the rutin poly(H-)sulfate salts of this invention may be administered in the form of conventional pharmaceutical compositions. Such compositions may be formulated so as to be suitable for oral or parenteral administration. The active ingredient may be combined in admixture with a pharmaceutically acceptable carrier, which carrier may take a wide variety of forms depending on the form of preparation desired for administration, i.e., oral or parenteral. The sulfate salts can be used in compositions such as tablets. Here, the principal active ingredient is mixed with conventional tabletting ingredients such as corn starch, lactose, sucrose, sorbitol, talc, stearic acid, magnesium stearate, dicalcium phosphate, gums, or similar materials as non-toxic pharmaceutically acceptable diluents or carriers. The tablets or pills of the novel compositions can be laminated or otherwise compounded to provide a dosage form affording the advantage of prolonged or delayed action or predetermined successive action of the enclosed medication. For example, the tablet or pill can comprise an inner dosage and an outer dosage component, the latter being in the form of an envelope over the former. The two components can be separated by an enteric layer which serves to resist disintegration in the stomach and permits the inner component to pass intact into the duodenum or to be delayed in release. A variety of materials can be used for such enteric layers or coatings, such materials including a number of polymeric acids or mixtures or polymeric acids with such materials as shellac, shellac and cetyl alcohol, cellulose acetate and the like. A particularly advantageous enteric coating comprises a styrene maleic acid copolymer together with known materials contributing to the enteric properties of the coating. The tablet of pill may be colored through the use of an appropriate non-toxic dye, so as to provide a pleasing appearance.

The liquid forms in which the novel compositions of the present invention may be incorporated for administration include suitable flavored emulsions with edible oils, such as, cottonseed oil, sesame oil, coconut oil, peanut oil, and the like, as well as elixirs and similar pharmaceutical vehicles. Sterile suspensions or solutions can be prepared for parenteral use. Isotonic preparations containing suitable preservatives are also desirable for injection use.

The term dosage form, as described herein, refers to physically discrete units suitable as unitary dosage for warm-blooded animal subjects, each unit containing a predetermined quantity of active component calculated to produce the desired therapeutic effect in association with the required pharamceutical diluent, carrier or vehicle. The specification for the novel dosage forms of this invention are indicated by characteristics of the active component and the particular therapeutic effect to be achieved or the limitations inherent in the art of compounding such an active component for therapeutic use in warm-blooded animals as disclosed in this specification. Examples of suitable oral dosage forms in accord with this invention are tablets, capsules, pills, powder packets, granules, wafers, cachets, teaspoonfuls, dropperfuls, ampules, vials, segregated multiples of any of the foregoing and other forms as herein described.

As indicated above, the novel compounds of this invention may be formulated with a wide variety of pharmaceutically acceptable carriers or diluents. Illustrative pharmaceutical compositions which are also suitable for this invention are given in Examples 7 to 24 of European Application No. 13470A, supra.

Thus, the novel compounds of the present invention, may be used in a method of inhibiting the complement system in a body fluid, such as blood serum, which comprises subjecting body fluid complement to the action of an effective complement inhibiting amount of rutin poly(H-)sulfate salt. The method of use aspect of this invention is further concerned with a method of inhibiting the complement system in a warm-blooded animal which comprises administering to said animal an effective complement inhibiting amount of a rutin poly(H-)sulfate salt. Body fluid can include blood, plasma, serum, synovial fluid, cerebrospinal fluid, or pathological accumulations of fluid such as pleural effusion, etc.

The rutin nona- and deca- (H-)sulfate salts of the present invention find utility as complement inhibitors in body fluids and as such may be used to ameliorate or prevent those pathological reactions requiring the function of complement and in the therapeutic treatment of warm--blooded animals having immunologic diseases such as rheumatoid arthritis, systemic lupus erythematosus, certain kinds of glomerulonephritis, certain kinds of autoallergic hemolytic anemia, certain kinds of platelet disorders and certain kinds of vasculitis. The instant compounds may also be used in the therapeutic treatment of warm-blooded animals having non-immunologic diseases such as paroxysmal nocturnal hemoglobinurea, hereditary angioneurotic edema (such as Suramin sodium, etc) and inflammatory states induced by the action of bacterial or lysosomal enzymes on the appropriate complement components as for example, inflammation following coronary occlusion. They may also be useful in the treatment of transplant rejection and as blood culture and transport mediums.

It will be understood that the present rutin

sulfate salts are solvated in varying degrees with water and ethanol.

CLAIMS:

1. A rutin poly(H-)sulfate of the formula:

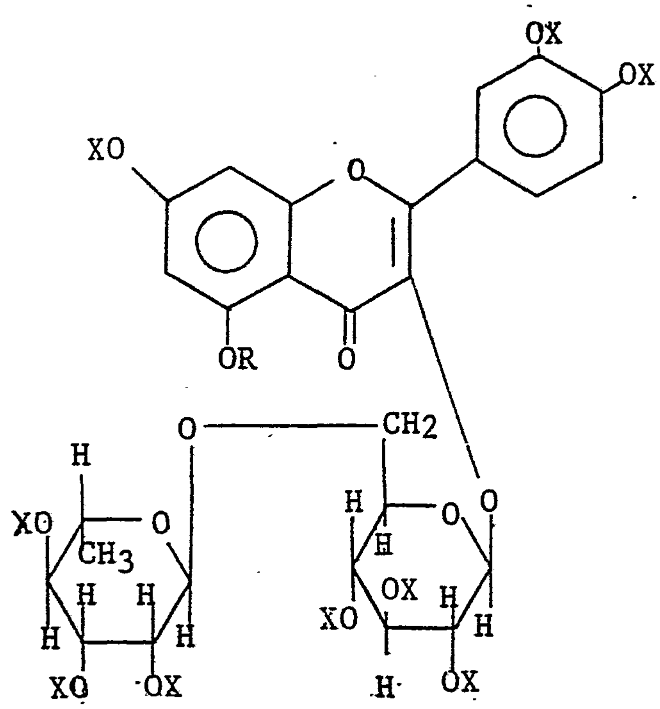

wherein each X is $-SO_3A$; R is hydrogen or $-SO_3A$; and A is a pharmaceutically acceptable salt cation.

2. The rutin poly(H-sulfate) according to Claim 1 wherein R is hydrogen, characterized in that the compound is free or substantially free of the rutin poly(H-sulfate) according to Claim 1 in which R is $-SO_3A$.

3. The rutin poly(H-sulfate) according to Claim 1 wherein R is $-SO_3A$, characterized in that the compound is free or substantially free of the rutin poly(H-sulfate) according to Claim 1 in which R is hydrogen.

4. Rutin deca(H-sulfate)sodium salt according to Claim 3.

5. Rutin nona(H-sulfate)sodium salt according to claim 2.

6. A method of inhibiting the complement system in a body fluid which comprises subjecting said body fluid to the action of an effective complement inhibiting amount of a rutin poly(H-)sulfate of the formula:

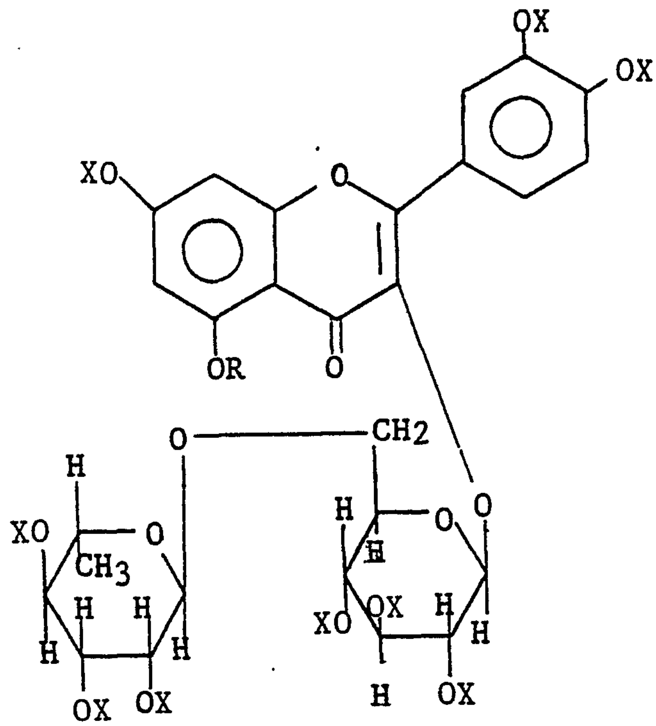

wherein each X is $-SO_3A$; R is hydrogen or $-SO_3A$; A is a pharmaceutically acceptable salt cation.

7. A method according to Claim 6, wherein the body fluid is blood serum.

8. A method according to Claim 6, wherein the rutin poly(H-)sulfate is rutin deca(H-sulfate)sodium salt free or substantially free of rutin nona(H-sulfate)sodium salt.

9. A method according to Claim 6, wherein the rutin poly(H-)sulfate is rutin nona(H-sulfate) sodium salt free or substantially free of rutin deca(H-sulfate)sodium salt.

10. A method of inhibiting the complement system in a warm-blooded animal which comprises administering to said animal an effective complement inhibiting amount of a rutin poly(H-)sulfate of the formula:

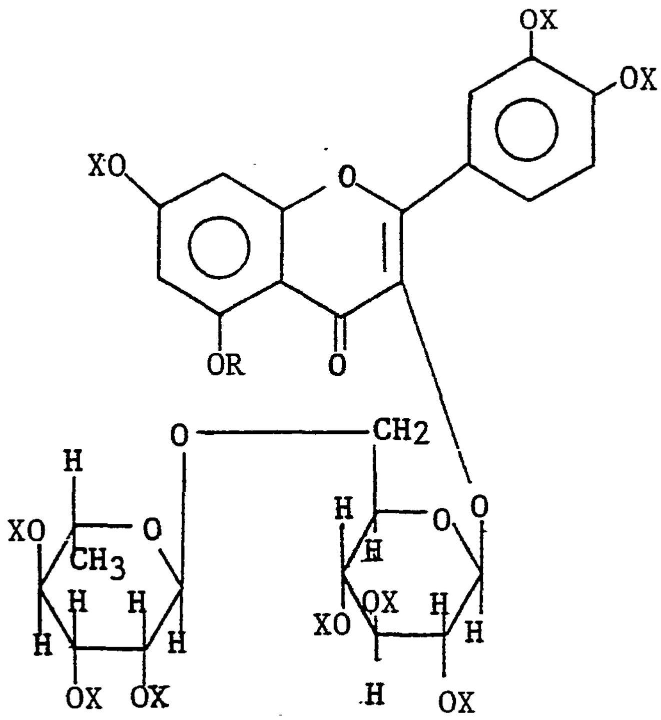

wherein each X is $-SO_3A$; R is hydrogen or $-SO_3A$; and A is a pharmaceutically acceptable salt cation.

11. A method according to Claim 10, wherein the rutin poly(H-)sulfate is administered internally.

12. A method according to Claim 10, wherein the rutin poly(H-)sulfate is administered topically.

13. A method according to Claim 10, wherein the rutin poly(H-)sulfate is administered periodontally in the oral cavity.

14. A method according to Claim 10, wherein the rutin poly(H-)sulfate is administered intra-articularly.

15. A method according to Claim 10, wherein the rutin poly(H-)sulfate is administered parenterally.

16. A method according to Claim 10, wherein the rutin poly(H-)sulfate is rutin deca(H-)sulfate sodium salt free or substantially free of rutin nona(H-)sulfate sodium salt.

17. A method according to Claim 10, wherein the rutin poly(H-)sulfate is rutin nona(H-)sulfate sodium salt free or substantially free of rutin deca(H-)sulfate sodium salt.

18. A method for the preparation of rutin deca

(H-)sulfate of the formula:

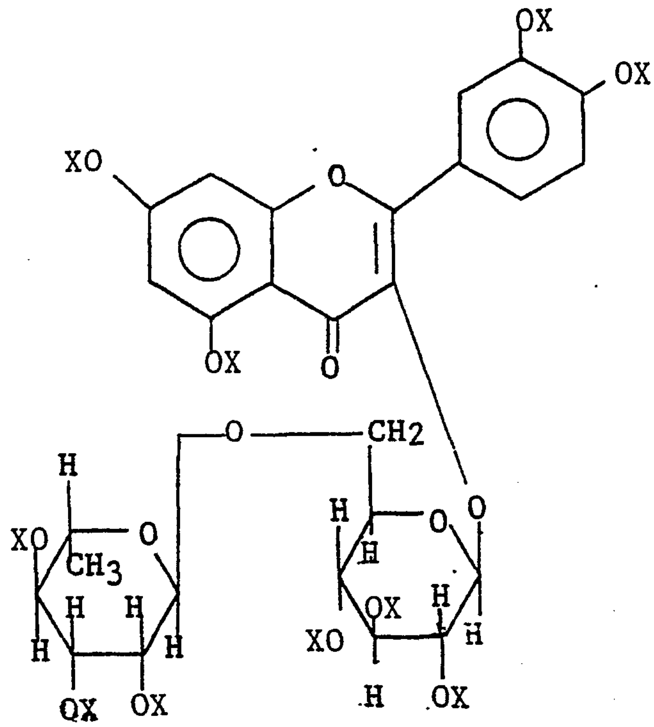

wherein each X is $-SO_3A$; and A is a pharmaceutically acceptable salt cation which process comprises the steps of:

(1) reacting rutin or a rutin poly(H-)sulfate having from one to nine sulfates in a suitable solvent with a triethylamine-sulfur trioxide complex at 50°-60°C:

(2) isolating the resulting triethylamine sulfate salt from acetone containing amine and cooled to below room temperature; and

(3) treating the isolated salt from step (2) with a pharmaceutically acceptable salt cation in aqueous solution.

19. A method according to Claim 17, wherein said solvent in step (1) is N,N-dimethylacetamide, and wherein the triethylamine sulfate salt is isolated in step (2) at about 0°C from acetone containing triethylamine as said amine.

20. A method according to Claim 17, wherein said pharmaceutically acceptable salt cation in step (3) is sodium.

0075626

21. A method for the preparation of rutin nona(H-)sulfate of the formula:

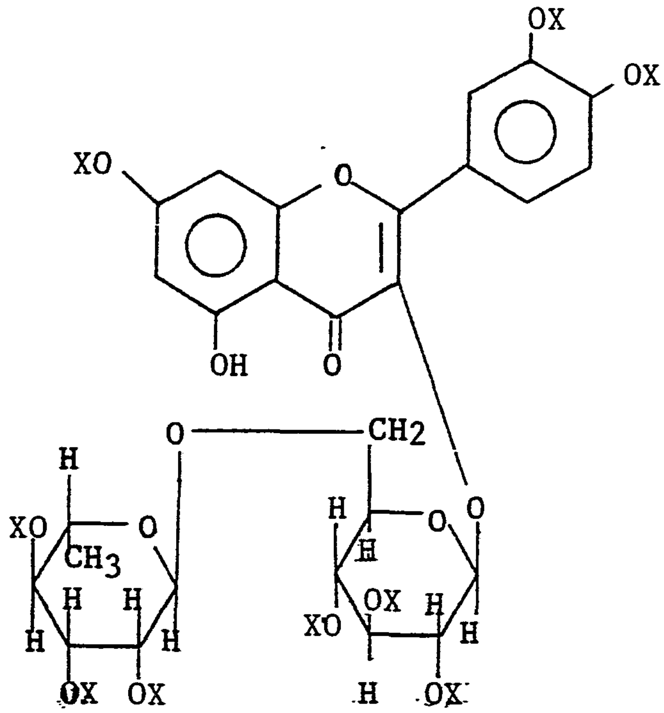

wherein each X is $-XO_3A$, and A is a pharmaceutically acceptable salt cation; which process comprises the steps of:

(1) reacting rutin or a rutin poly(H-)sulfate having from one to eight sulfates in a suitable solvent with a triethylamine-sulfur trioxide complex at 50°-90°C;

(2) isolating the resulting triethylamine sulfate salt from acetone at ambient temperatures; and

(3) treating the isolated salt from step (2) with a pharmaceutically acceptable salt cation in aqueous solution.

22. A method according to Claim 20, wherein said solvent in step (1) is N,N-dimethylacetamide, and wherein said pharmaceutically acceptable salt cation in step (3) is sodium.

LLOYD WISE, TREGEAR & (
NORMAN HOUSE 105-109 STR

European Patent Office

# PARTIAL EUROPEAN SEARCH REPORT

which under Rule 45 of the European Patent Convention shall be considered, for the purposes of subsequent proceedings, as the European search report

0075626

Application number

EP 81 30 4492

## DOCUMENTS CONSIDERED TO BE RELEVANT

| Category | Citation of document with indication, where appropriate, of relevant passages | Relevant to claim | CLASSIFICATION OF THE APPLICATION (Int. Cl.[3]) |
|---|---|---|---|
| D,X | EP - A - 0 013 470 (AMERICAN CYANAMID CO.)<br>* pages 1-3, claims *<br>--------- | 1-22 | C 07 H 17/06<br>A 61 K 31/70 |

TECHNICAL FIELDS SEARCHED (Int. Cl.[3])

C 07 H 17/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-5,18-22

Claims searched incompletely: 6-17

Claims not searched:

Reason for the limitation of the search: Method for treatment of the human or animal body by surgery or therapy (See article 52(4) of the European Patent Convention)

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

| Place of search | Date of completion of the search | Examiner |
|---|---|---|
| The Hague | 11th May 1982 | VERHULST |

EPO Form 1505.1 06.78